# EUROPEAN PATENT APPLICATION

(11) **EP 1 006 112 A1**
(43) Date of publication of application: **07.06.2000**
(21) Application number: 99309629.6
(22) Date of filing: 01.12.1999
(51) Int. Cl.: C07D 401/12, C07D 401/06, A61K 31/4412, A61P 25/00, C07D 401/14

(54) **3-Hydroxy-2(1H)-pyridinone or 3-hydroxy-4(1H)-pyridinone derivatives useful as reactive oxygen species (ROS) scavengers**

(30) Priority: 01.12.1998 GB 9826357; 23.04.1999 GB 9909496
(71) Applicant: Cerebrus Pharmaceuticals Limited, Winnersh, Wokingham RG41 5UA (GB)
(72) Inventor: Bebbington, David, C/o Cerebrus Pharmac. Ltd., Winnersh, Wokingham RG41 5UA (GB); Gaur, Suneel, C/o Cerebrus Pharmaceuticals Ltd., Winnersh, Wokingham RG41 5UA (GB)
(74) Representative: Howard, Paul Nicholas

(57) **Abstract**

A compound of the formula (1): 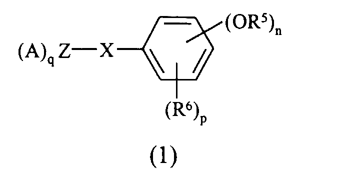 wherein A is 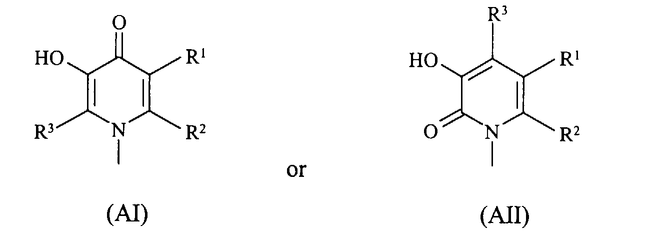
wherein R¹, R² and R³ are independently selected from H and alkyl;
wherein X is O, S, NR⁴ or a direct bond, wherein R⁴ is H or alkyl;
wherein Z is a hydrocarbyl chain comprising from 2 to 10 carbon atoms and one or more carbon-carbon double bonds, the hydrocarbyl chain also being optionally interrupted by one or more heteroatom(s) and optionally substituted by one or more oxo substituent(s);
wherein q is 1, 2 or 3;
wherein if q is 2 or 3, then each A can be the same or different;
wherein the or each R⁵ is independently selected from H and alkyl;
wherein the or each R⁶ is independently selected from alkyl;
wherein n is 1 to 5;
wherein p is 0 to 4; and
wherein the sum of n and p is less than 6,
or a pharmaceutically acceptable salt thereof, and the use thereof in therapy, particularly for the treatment of a condition associated with oxidative stress, such as oxidative damage of the central nervous system or an acute or chronic neurological disorder such as traumatic brain injury, spinal cord injury, cerebral tumour, subharrachnoid haemorrage/cerebral vasospasm, cerebral ischaemia, stroke (ischaemic or haemorragic), Alzheimer's disease, Huntington's Disease, Parkinson's Disease, Friedrich's ataxia, motor neuron disease or multiple sclerosis.

## Description

The present invention relates to compounds containing both ortho-hydroxypyridinone and oxygenated aryl (including heteroaryl) functionalities, which possess the dual ability to chelate iron and scavenge reactive oxygen species (ROS). In particular, the invention relates to specific compounds containing a 3-hydroxy-4(1*H*)-pyridinone or a 3-hydroxy-2(1*H*)-pyridinone iron chelating moiety as well as a phenolic or oxy-substituted indolic antioxidant moiety. The present invention also relates to the synthesis of such compounds, to pharmaceutical preparations comprising such compounds and to the use of such compounds in the treatment and prophylaxis of conditions associated with oxidative stress, particularly oxidative damage to the central nervous system.

One particularly relevant example of a condition involving oxidative damage to the central nervous system, which can be treated with compounds of the present invention, is stroke. Stroke is the third leading cause of death in major industrialised countries and the commonest cause of permanent disability (Hunter *et al.,* Trends in Pharmacological Sciences, 1995, 16, 123-128). Each year, in the US and Europe, approximately 1 million people suffer acute stroke (Dorman *et al.,* CNS Drugs, 1996, 5, 457-474). Between 25% and 35% of these patients die within the first three weeks, and of the survivors 25% to 50% will be totally dependant on family or institutional care for the rest of their lives. The incidence of stroke increases with age, roughly doubling with each passing decade, with 30% of persons aged over 65 years being affected (Babikian *et al.,* Cerebrovascular disease in the elderly. In Clinical Neurology of Aging, Eds Albert M.L. and Knoefel J.E., OUP, New York, 1994). These statistics translate into an annual incidence of 0.1 to 0.2% in the US and Europe, with the world-wide market for stroke estimated to be worth $3 billion in 1995 and projected to rise to $10 billion in 2005.

Stroke is defined as an interruption of the blood flow to the brain or leakage of blood out of the brain resulting in oxygen deprivation (ischaemia) and subsequent neuronal cell death. Strokes can be divided into two classes, ischaemic and haemorragic. The former accounts for approximately 83% of all strokes and is caused by thrombosis (65%) and/or detachment of a previously formed clot (embolus, 18%). Haemorrhagic strokes, which account for the remaining 17% of all strokes, can be subdivided into subarachnoid haemorrhage (7%) and cerebral haemorrhage (10%).

Markers of oxidative damage have been detected in the brains of ischaemic animals, and a variety of antioxidant molecules have been demonstrated to be neuroprotective in ischaemic stroke models. This provides conclusive evidence that cerebral ischaemia leads to the production of reactive free radicals. Defined as a chemical species containing one or more unpaired electrons, and capable of independent existence, free radicals are highly destructive towards cellular membrane lipids, DNA and proteins. This "oxidative-modification" of cellular components ultimately leads to a loss of cell function. One example of this oxidative process is lipid peroxidation (LP), a process which increases membrane fluidity leading to failure of normal membrane potential, disturbance of calcium homeostasis and transmembrane signalling, with the ultimate result of neuronal cell death.

A combination of the brains high-energy demand and subsequent high rate of oxygen consumption, with its limited endogenous antioxidant defences (superoxide dismutase (SOD), glutathione peroxidase (GSPx), and catalase) makes the brain very susceptible to free radical attack. Additionally, neuronal cell membranes, being rich in polyunsaturated fatty acids, are especially vulnerable to oxidative modification. The brains vulnerability to free radical attack is further exacerbated by relatively high levels of iron in the brain. Iron is the fundamental catalyst in the production of the hydroxyl radical (^{·}OH) (Fenton and Haber-Weiss Reactions), reportedly the most destructive of all free radicals (Palmer, C., Metals and Oxidative Damage in Neurological Disorders, Ed. Connor, Plenum Press, New York, 1997, pp 205-236).

Exposure to free radicals is a natural consequence of aerobic respiration, to which the human body possesses a variety of natural antioxidant mechanisms. However, during pathological conditions such as stroke homeostatic mechanisms break down, and the balance between the generation of free radicals and natural antioxidant defences is shifted, resulting in a state of oxidative stress (Beal M.F., Ann. Neurol., 1995, 31, 119-130; Gerlach *et al.,* J. Neurochem., 1994, 63, 793-807).

In animal models of stroke, supplementation of antioxidant defences with exogenous antioxidant molecules has resulted in neuroprotection, as assessed both histologically and behaviourally (Hall E.H., Metals and Oxidative Damage in Neurological Disorders, *supra,* pp 325-339). Furthermore, transgenic animals overexpressing SOD have been demonstrated to be more resistant to cerebral ischaemia than their wild type littermates (Chan *et al.,* Ann. N.Y. Acad. Sci., 1994, 738, 93-103).

The iron chelator deferiprone (1,2-dimethyl-3-hydroxy-4(1*H*)-pyridinone) has also been shown to possess antioxidant properties. For example the use of deferiprone to inhibit free radical formation has been disclosed by Kontochiorghes *et al.* (Free Rad. Res. Comms., 1986, 2, 115-124) and by Mostert *et al.* (Free Rad. Res. Comms., 1987, 3, 379-388). The use of deferiprone in conjunction with an antioxidant is also disclosed in WO 94/03169 for use in the treatment of sickle cell disease, and by Antonius *et al.* (Circulation, 1989, 80, 158-164) for use in the prevention of postischemic cardiac injury. Deferiprone has recently been in clinical trials as a potential treatment for iron overload in thalassemia major, and has also been disclosed for the treatment of parasitic infections, anemia and Alzheimer's disease.

There are many cellular systems known to be inappropriately activated or regulated as a result of oxygen starvation to the brain (e.g. glutamate receptors, voltage dependent ion channels). A major consequence of this is a loss of calcium homeostasis and inappropriate enzyme activation via several routes. Generation of free radicals is a common biochemical end point to many of the processes that are inappropriately regulated following cerebral ischaemia (Dorman *et al., supra,* Hall E.H. *supra;* Patt *et al.,* J. Pediatric Surg., 1990, 25, 224-227). Hence intervention "down stream" with an antioxidant molecule at a point where many of these processes converge, is considered to be strategically sound owing to a universal applicability to many intracellular processes.

Based on the above rationale, a low molecular weight molecule designed to simultaneously trap radicals and chelate iron is a novel, scientifically relevant approach towards the treatment of conditions associated with oxidative stress, in particular cerebral ischaemia/stroke.

There have been three related reports describing molecular entities with dual iron chelating and anti-oxidant capabilities. The first report, Sato *et al* (Bioconjugate Chem., 1995, 6, 249-54), describes Cu,Zn-superoxide dismutase and desferrioxamine conjugated via polyoxyethylene. This high molecular weight conjugate was not used to show protection against oxidative stress *in vitro,* nor was it investigated for its effectiveness in *in vivo* models of oxidative stress. The second report, Rojanasakul *et al* (Biochim. Biophys. Acta, 1996, 1315(1), 21-8), describes a transferrin-catalase conjugate which gave increased protection to cells from oxidative stress as a result of its increased uptake in to cells *via* the transferrin receptor. The paper suggests the potential therapeutic use of the "...conjugate for the treatment of pathological processes in the lung". This high molecular weight conjugate was not investigated for its effectiveness in *in vivo* models of oxidative stress. The third report, Tilbrook *et al* (WO 9825905), claims compounds containing iron chelator units linked to a group containing reducing -SH groups for the treatment of Alzheimer's disease and related neurodegenerative diseases. The compounds were not investigated for their effectiveness in *in vivo* models of oxidative stress.

Currently there are only two recognised forms of treatment available for stroke victims. The first, Altepase^{®} (recombinant tissue plasminogen activator, rTPA) is a clot busting drug only suitable for cerebral thrombosis. Therapeutic thrombolysis can, however, be complicated by a) systemic haemorrhage, b) intracerebral haemorrhage, c) distal embolism of a partially digested clot leading to secondary infarction and d) cerebral oedema secondary to reperfusion injury. It is, therefore, necessary to exclude the possibility of haemorrhagic stroke by computerised tomographic (CT) scanning of patients before administering Alteplase. The second recognised treatment, carotid endarterectomy is a surgical procedure for unblocking the carotid artery. However, both treatments have the potential to exacerbate and complicate the original injury, and neither treatment is neuroprotective nor universal for all types of stroke. A third treatment, the antioxidant idebenone is licensed for the treatment of stroke in Japan. There is therefore a large unmet medical need for an effective neuroprotective compound for the treatment of stroke.

It is an object of this invention to provide compounds which, unlike the current therapies used for the treatment of stroke, protect against damage due to reperfusion injury, and are neuroprotective. Such compounds are potentially useful for all types of stroke. It is a further object of this invention to provide compounds which may be used in the treatment of oxidative stress generally and particularly oxidative damage to the central nervous system.

According to the present invention there is provided a compound of the formula (1): 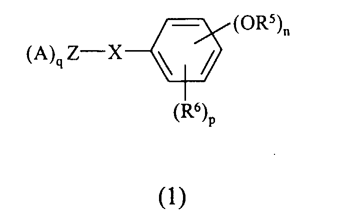 wherein A is 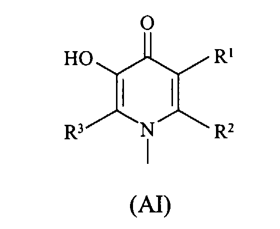 or 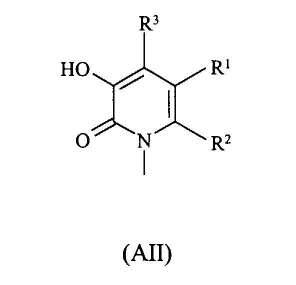
wherein R¹, R² and R³ are independently selected from H and alkyl;
wherein X is O, S, NR⁴ or a direct bond, wherein R⁴ is H or alkyl;
wherein Z is a hydrocarbyl chain comprising from 2 to 10 carbon atoms and one or more carbon-carbon double bonds, the hydrocarbyl chain also being optionally interrupted by one or more heteroatom(s) and optionally substituted by one or more oxo substituent(s);
wherein q is 1, 2 or 3;
wherein if q is 2 or 3, then each A can be the same or different;
wherein the or each R⁵ is independently selected from H and alkyl;
wherein the or each R⁶ is independently selected from alkyl;
wherein n is 1 to 5;
wherein p is 0 to 4; and
wherein the sum of n and p is less than 6,
or a pharmaceutically acceptable salt thereof.

It has been found that the compounds of the present invention which have a combined antioxidant and iron chelating activity can be used for treating oxidative stress, particularly oxidative damage to the central nervous system. It has further been found that the compounds of the present invention are surprisingly more effective, especially at low concentrations *in vitro,* than the simultaneous use of the separate ortho-hydroxypyridone iron chelating compound and the oxygenated aryl (including heteroaryl) antioxidant compound.

As used herein, the term "alkyl" means a branched or unbranched, cyclic or acyclic, saturated or unsaturated (e.g. alkenyl or alkynyl) hydrocarbyl radical which may be substituted or unsubstituted. Where cyclic, the alkyl group is preferably C₃ to C₁₂, more preferably C₅ to C₁₀, more preferably C₅ to C₇. Where acyclic, the alkyl group is preferably C₁ to C₁₀, more preferably C₁ to C₆, more preferably methyl, ethyl, propyl (n-propyl or isopropyl), butyl (n-butyl, isobutyl or tertiary-butyl) or pentyl (including n-pentyl and isopentyl), more preferably methyl. It will be appreciated therefore that the term "alkyl" as used herein includes alkyl (branched or unbranched), substituted alkyl (branched or unbranched), alkenyl (branched or unbranched), substituted alkenyl (branched or unbranched), alkynyl (branched or unbranched), substituted alkynyl (branched or unbranched), cycloalkyl, substituted cycloalkyl, cycloalkenyl, substituted cycloalkenyl, cycloalkynyl and substituted cycloalkynyl.

As used herein, the term "aryl" means a substituted or unsubstituted carbocyclic aromatic group, such as phenyl or naphthyl, or a substituted or unsubstituted heteroaromatic group containing one or more, preferably one, heteroatom, such as pyridyl, pyrrolyl, furanyl, thienyl, thiazolyl, isothiazolyl, oxazolyl, isoxazolyl, oxadiazolyl, thiadiazolyl pyrazolyl, imidazolyl, triazolyl, pyrimidinyl pyridazinyl, pyrazinyl, triazinyl, indolyl, indazolyl, quinolyl, quinazolyl, benzimidazolyl, benzothiazolyl, benzisoxazolyl and benzisothiazolyl.

As used herein, the term "carbocyclic" refers to a ring system in which all the ring atoms are carbon atoms.

The alkyl and aryl groups may be substituted or unsubstituted. Where substituted, there will generally be 1 to 3 substituents present, preferably 1 substituent. Substituents may include:
carbon-containing groups such as
   alkyl,
   aryl,
   arylalkyl (e.g. substituted and unsubstituted phenyl, substituted and unsubstituted benzyl);
halogen atoms and halogen-containing groups such as
   - haloalkyl: (e.g. trifluoromethyl);
oxygen-containing groups such as
   - alcohols: (e.g. hydroxy, hydroxyalkyl, aryl(hydroxy)alkyl),
   - ethers: (e.g. alkoxy, aryloxy, alkoxyalkyl, aryloxyalkyl),
   - aldehydes: (e.g. carboxaldehyde),
   - ketones (e.g. alkylcarbonyl, alkylcarbonylalkyl, arylcarbonyl, arylalkylcarbonyl, arylcarbonylalkyl),
   - acids: (e.g. carboxy, carboxyalkyl),
   acid derivatives such as esters (e.g. alkoxycarbonyl, alkoxycarbonylalkyl, alkylcarbonyloxy, alkylcarbonyloxyalkyl), amides (e.g. aminocarbonyl, mono- or di-alkylaminocarbonyl, aminocarbonylalkyl, mono- or di-alkylaminocarbonylalkyl, arylaminocarbonyl),
   - carbamates: (e.g. alkoxycarbonylamino, aryloxycarbonylamino, aminocarbonyloxy, mono- or di-alkylaminocarbonyloxy, arylaminocarbonyloxy)
   - and ureas: (e.g. mono- or di-alkylaminocarbonylamino or arylaminocarbonylamino);
and nitrogen-containing groups such as
   - amines: (e.g. amino, mono- or di-alkylamino, aminoalkyl, mono- or di-alkylaminoalkyl).

As used herein, the term "alkoxy" means alkyl-O-. Alkoxy substituent groups or alkoxy-containing substituent groups may be substituted by one or more alkyl groups.

As used herein, the term "halogen" means a fluorine, chlorine, bromine or iodine radical, preferably a fluorine or chlorine radical.

In a preferred embodiment, the alkyl groups are either unsubstituted or substituted by substitution of a hydrogen atom with a group selected from OR⁷, OCOR⁷, COOR⁷, NHR⁷, NHCOR⁷ and CONHR⁷ wherein R⁷ is H or alkyl.

As used herein, the term "oxy-substituted" is a general term used to describe substitution by a hydroxy and/or an alkoxy group.

As used herein, the term "heteroatom" means an atom other than a carbon atom, such as nitrogen, oxygen or sulphur.

As used herein, the term "oxo substituent" means an oxygen atom joined to a carbon atom or a heteroatom, such as sulphur, in the hydrocarbyl chain by a double bond.

As used herein, the term "interrupted by one or more heteroatom(s)" means that the or each heteroatom may be positioned at any position along the hydrocarbyl chain including at either end of the chain.

As used herein, the term "interrupted by one or more group(s)", the group(s) being selected from O, S, SO, SO₂, SO₂NH, NHSO₂, NH, NHCO, CONH, NHCONH, NHCOO, OCONH, OCO and COO, means that the or each group may be positioned at any position along the hydrocarbyl chain including at either end of the chain.

As used herein, the term "pharmaceutically acceptable salt" means any pharmaceutically acceptable salt of the compound of formula (1). Salts may be prepared from pharmaceutically acceptable non-toxic acids and bases including inorganic and organic acids and bases. Such acids include acetic, benzenesulfonic, benzoic, camphorsulfonic, citric, ethenesulfonic, dichloroacetic, fumaric, gluconic, glutamic, hippuric, hydrobromic, hydrochloric, isethionic, lactic, maleic, malic, mandelic, methanesulfonic, mucic, nitric, pamoic, pantothenic, phosphoric, succinic, sulfuric, tartaric, oxalic, p-toluenesulfonic and the like. Particularly preferred are hydrochloric, hydrobromic, phosphoric, sulfuric and methanesulfonic acids, and most particularly preferred is the methanesulfonate salt. Acceptable base salts include alkali metal (e.g. sodium, potassium), alkaline earth metal (e.g. calcium, magnesium) and aluminium salts.

According to the present invention, the or each A is independently linked to the chain Z at any atom of the chain Z. It is preferred that the or each A is independently linked to the chain Z at a carbon, nitrogen or oxygen atom of the chain Z.

According to the present invention, when A is AI, preferably R² and R³ are independently selected from H, unsubstituted alkyl, CH₂OR⁷, CH₂OCOR⁷, COOR⁷, CH₂NHR⁷, CH₂NHCOR⁷ and CONHR⁷ wherein R⁷ is H or alkyl. Preferably, R¹ is selected from H and unsubstituted alkyl. More preferably, R¹, R² and R³ are independently selected from H and unsubstituted alkyl. More preferably, R¹ and R² are H and R³ is unsubstituted alkyl. It is further preferred that R³ is methyl.

According to the present invention, when A is All, preferably R¹, R² and R³ are independently selected from H, unsubstituted alkyl, CH₂OR⁷, CH₂OCOR⁷, COOR⁷, CH₂NHR⁷, CH₂NHCOR⁷ and CONHR⁷ wherein R⁷ is H or alkyl. Preferably, R¹, R² and R³ are independently selected from H and unsubstituted alkyl. More preferably, R¹, R² and R³ are H.

Preferably, the present invention provides a compound wherein A is AI.

Preferably the present invention provides a compound wherein q=1.

In a preferred embodiment, Z is a hydrocarbyl chain comprising from 2 to 10 carbon atoms and one or more carbon-carbon double bonds, the hydrocarbyl chain also being optionally interrupted by one or more group(s) selected from O, S, SO, SO₂, SO₂NH, NHSO₂, NH, NHCO, CONH, NHCONH, NHCOO, OCONH, OCO and COO.

Preferably, the present invention provides compounds wherein Z comprises from 2 to 8 carbon atoms.

In a further preferred embodiment, Z is (CH₂)ₖWY wherein k is 0 to 10, wherein W is selected from O, S, SO, SO₂, SO₂NH, NHSO₂, NH, NHCO, CONH, NHCONH, NHCOO, OCONH, OCO, COO and a direct bond; wherein Y is a hydrocarbyl chain comprising from 2 to 10 carbon atoms and one or more carbon-carbon double bond(s); and wherein the chain (CH₂)ₖWY comprises from 2 to 10 carbon atoms. In this embodiment, it is preferred that when k is 0, W is selected from O, NH, NHCO, NHCONH, NHCOO, OCONH and a direct bond; and when k is 1, W is selected from SO, SO₂, SO₂NH, CONH, COO and a direct bond.

Preferably, the present invention provides compounds wherein W is selected from O, NH, CONH, NHCO and a direct bond.

Preferably, the present invention provides compounds wherein k is 1, 2, 3 or 4. Preferably, the hydrocarbyl chain Y comprises 2, 3, 4 or 5 carbon atoms and preferably 3, 4 or 5 carbon atoms.

Preferably the present invention provides compounds wherein Z contains one carbon-carbon double bond.

According to the present invention, the chain Z may be branched or unbranched, optionally substituted by one or more alkyl groups, and may be cyclic. Preferably, Z is an unbranched chain. It will be appreciated that where q=1, Z is a di-valent hydrocarbyl chain; where q=2, Z is a tri-valent hydrocarbyl chain; and where q=3, Z is a tetra-valent hydrocarbyl chain.

As used herein to describe the hydrocarbyl chain Z, the term "cyclic" means either that Z may comprise a cyclic hydrocarbyl group of from 3 to 10 carbon atoms, preferably 5, 6 or 7 carbon atoms; or that where R⁵ or R⁶ is alkyl, a cyclic group is present as a result of cyclisation of the alkyl group of R⁵ or R⁶ onto Z; or that, where X is NR⁴ and R⁴ is alkyl, a cyclic group is present as a result of cyclisation of R⁴ onto Z; or that a cyclic group is present as a result of cyclisation of Z onto the phenyl ring shown in formula (1) to form a fused ring system. It is preferred that a cyclic group formed as a result of cyclisation of R⁴, R⁵ or R⁶ onto Z, or as a result of cyclisation of Z onto the phenyl ring shown in formula (1), is a 5, 6 or 7-membered ring.

In a preferred embodiment of the present invention, Z is cyclised onto the phenyl ring shown in formula (1).

According to the present invention, X is preferably NR⁴ or a direct bond. Most preferably, X is NR⁴.

In a preferred embodiment of the present invention, where X is NR⁴ the carbon-carbon double bond of the group Z is immediately adjacent the NR⁴ group in the chain defined as Z-X. In a preferred embodiment, R⁴ is H.

According to the present invention, where X is NR⁴ and R⁴ is alkyl, R⁴ may be cyclized onto the chain Z.

According to the present invention, R⁵ is preferably selected from H and C₁₋₁₀ alkyl. In one embodiment of the invention at least one R⁵ is H. Preferably, when X is not NH, at least one R⁵ is H.

Preferably, the present invention provides compounds wherein n is 1 to 3, more preferably n is 1 or 2, most preferably n is 1.

According to the present invention, preferably OR⁵ is positioned in the ortho or para position in the ring with respect to X. More preferably, OR⁵ is positioned in the para position in the ring with respect to X.

According to the present invention, the or each R⁶ is preferably independently selected from C₁₋₁₀ alkyl, more preferably from C₁₋₄ alkyl, more preferably from methyl, isopropyl and t-butyl.

Preferably, the present invention provides compounds wherein p is 0, 1, 2 or 3.

According to the present invention, when p is 1 or 2, alkyl groups represented by R⁶ is/are preferably positioned ortho to OR⁵, preferably in the meta-position of the ring with respect to X to give, for example, a compound of formula:-

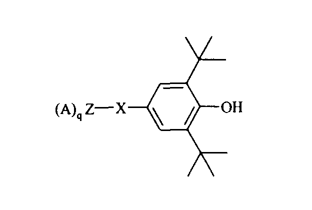

According to the present invention, when p is 3, alkyl groups represented by R⁶ are preferably positioned to give, for example where three R⁶ are methyl, a compound of formula:-

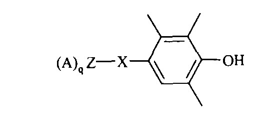

In an embodiment of the present invention, where R⁵ or R⁶ are alkyl, the alkyl group of R⁵ or R⁶ can be cyclized onto a carbon atom or a nitrogen atom of the chain defined as Z to form a ring.

In a preferred embodiment of the invention, X is O, S or NR₄, preferably NR⁴, and more preferably NH, the carbon-carbon double bond of the chain Z is immediately adjacent the group X in the chain defined as Z-X, and Z is cyclized onto the phenyl ring shown in formula (1) to give a 5 or 6 membered heterocyclic ring, preferably a 5-membered ring, containing a carbon-carbon double bond.

According to a further aspect of the present invention there is provided a method of preparing the compounds of the present invention. The compounds of the present invention may be prepared using standard synthetic chemistry.

A general method for synthesis of compounds where q = 1 and A is a 3-hydroxy-4(1*H*)-pyridinone moiety comprises condensation of the primary amine of the respective antioxidant unit (3) with a 3-benzyloxy-4-pyrone (2) (in the presence of base, for example NaOH in a MeOH:water solution), followed by removal of the benzyl protecting group (with for example H₂ and Pd/C), as illustrated in Reaction Scheme 1. (For the condensation of 3-benzyloxy-2-methyl-4-pyrone with simple primary amines, see Dobbin *et al.,* J. Med. Chem., 1993, 36, 2448-2458).

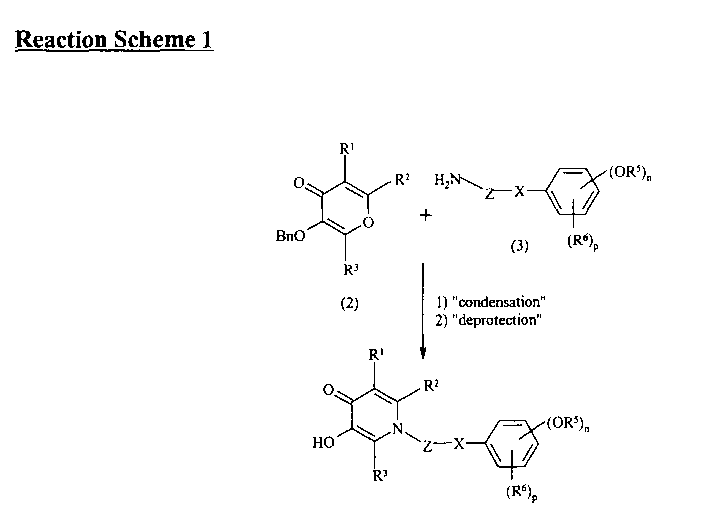

The primary amines, of the respective antioxidants, (3) can be prepared using standard synthetic chemistry.

A general method for synthesis of compounds where q = 1 and A is a 3-hydroxy-2(1*H*)-pyridinone moiety comprises N-alkylation of a 3-methoxy-2(1*H*)-pyridinone (4) with the halide of the respective antioxidant unit (5) (or an alternative electrophilic alkylating derivative of the antioxidant unit; for example, mesylate or tosylate), followed by removal of the methyl protecting group (with for example BBr₃), as illustrated in Reaction Scheme 2. (For the alkylation of 3-methoxy-2(1*H*)-pyridinone with simple alkyl iodides, see Dobbin *et al.,* J. Med. Chem., 1993, 36, 2448-2458).

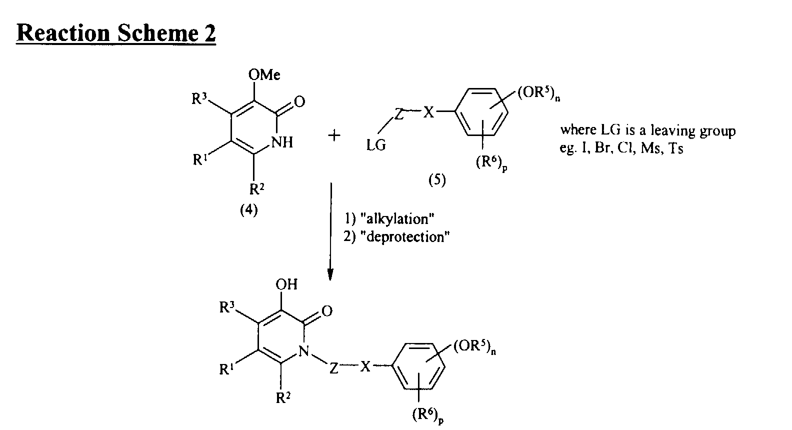

The primary halides, (mesylates and tosylates) of the antioxidants, (5) can be prepared using standard synthetic chemistry.

A specific method for the synthesis of compounds where q = 1, A is a 3-hydroxy-4-(1*H*)-pyridinone moiety and Z is (CH₂)ₖWY where W is a CONH, COO, SO₂NH or SO₂O unit (for example, the compound of Example 1) comprises coupling the acid derivative (carboxylic or sulfonic) of the protected hydroxy pyridinone (6) with the primary amine or alcohol of the respective antioxidant unit (7) using standard coupling techniques (for example via acid activation as a succinimdyl ester, or with coupling reagents such as EDCI), followed by removal of the benzyl protecting group, as illustrated in Reaction Scheme 3. (For the coupling of a compound of the type (6), where k is 2 and Q is CO, to simple amines see Dobbin *et al.* J. Med. Chem., 1993, 36, 2448-58).

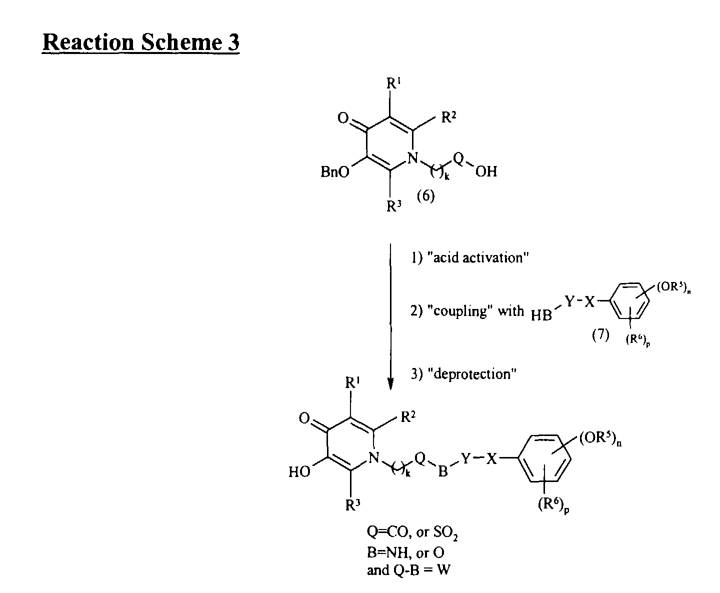

The acid derivatives (carboxylic or sulfonic) of the protected hydroxy pyridinone (6) can be prepared using standard synthetic chemistry. (For the synthesis of (6), where R¹ and R² are H, R³ is methyl, k is 2 and 3 and Q is CO, see Dobbin *et al.* J. Med. Chem., 1993, 36, 2448-58).

A specific method for the synthesis of compounds where q = 1, A is 3-hydroxy-2-(1*H*)-pyridinone moiety and Z is (CH₂)ₖWY where W is a CONH, COO, SO₂NH or SO₂O unit (for example, the compound of Example 3) comprises coupling of the acid derivative (carboxylic or sulfonic) of the protected hydroxy pyridinone (8) with the primary amine or alcohol of the respective antioxidant unit (7) using standard coupling techniques (for example via acid activation as a succinimdyl ester, or with coupling reagents such as EDCI), followed by removal of the benzyl protecting group, as illustrated in Reaction Scheme 4. (For the coupling of a compound of the type (8), where k is 1 and Q is CO, to simple amines see Streater *et al.* J. Med. Chem., 1990, 33, 1747-1755).

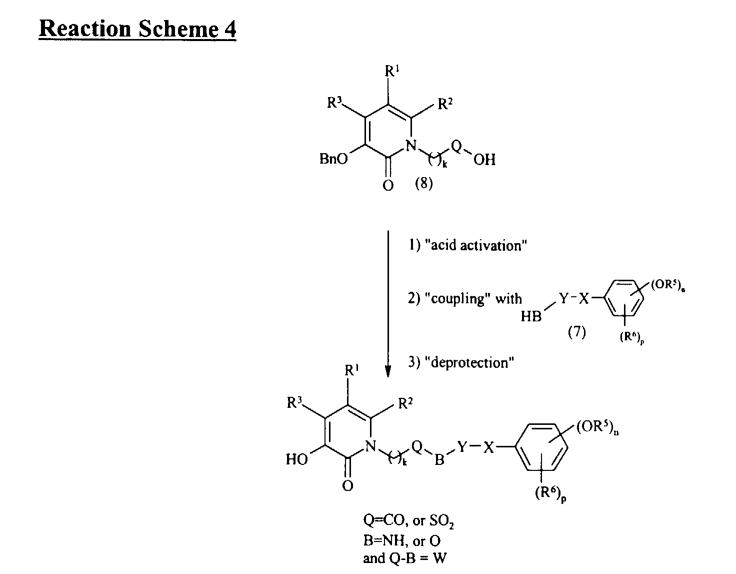

The acid derivatives (carboxylic or sulfonic) of the protected hydroxy pyridinone (8) can be prepared using standard synthetic chemistry. (For the synthesis of (8), where R¹, R² and R³ are H, k is 1 and Q is CO, see Streater *et al.* J. Med. Chem., 1990, 33, 1747-1755).

The primary amines and alcohol's, of the respective antioxidants, (7) can be prepared using standard synthetic chemistry.

A specific method for the synthesis of compounds where q = 1, A is a 3-hydroxy-4-(1*H*)-pyridinone moiety and Z is (CH₂)ₖWY where W is a NHCO, OCO, NHSO₂ or OSO₂ unit comprises coupling the primary amine or alcohol derivative of the protected hydroxy pyridinone (9) with the carboxylic or sulfonic acid derivative of the respective antioxidant unit (10) using standard coupling techniques (for example via acid activation as a succinimdyl ester, or with coupling reagents such as EDCI), followed by removal of the benzyl protecting group, as illustrated in Reaction Scheme 5. (For the coupling of (9), where R¹ and R² are H and R³ is methyl, k is 3 and B is NH, to simple acid derivatives see Miller *et al.* Synth. Comm., 1995, 25, 3247-53).

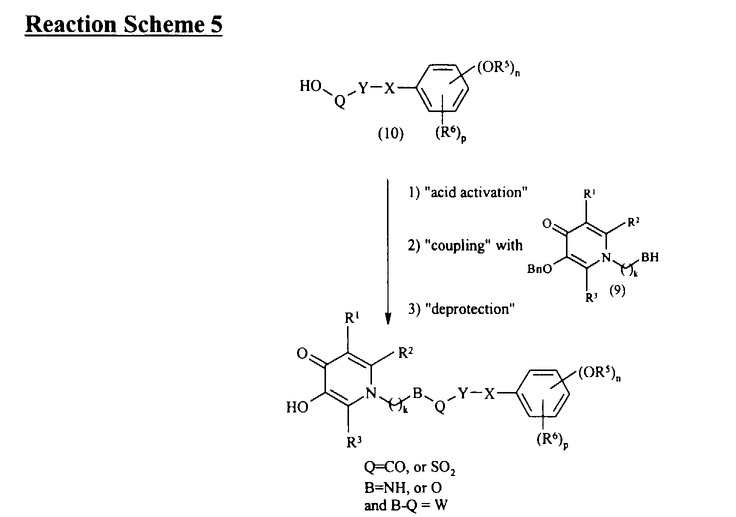

The primary amine or alcohol of the protected hydroxy pyridinone (9) can be prepared using standard synthetic chemistry. (For the synthesis of (9), where R¹ and R² are H and R³ is methyl, k is 2 and 3 and B is NH and O, see Dobbin *et al.* J. Med. Chem., 1993, 36, 2448-58).

A specific method for the synthesis of compounds where q = 1, A is a 3-hydroxy-2-(1*H*)-pyridinone moiety and Z is (CH₂)ₖWY where W is a NHCO, OCO, NHSO₂ or OSO₂ comprises coupling the primary amine or alcohol derivative of the protected hydroxy pyridinone (11) with the carboxylic or sulfonic acid derivative of the respective antioxidant unit (10) using standard coupling techniques (for example via acid activation as a succinimdyl ester, or with coupling reagents such as EDCI), followed by removal of the benzyl protecting group, as illustrated in Reaction Scheme 6.

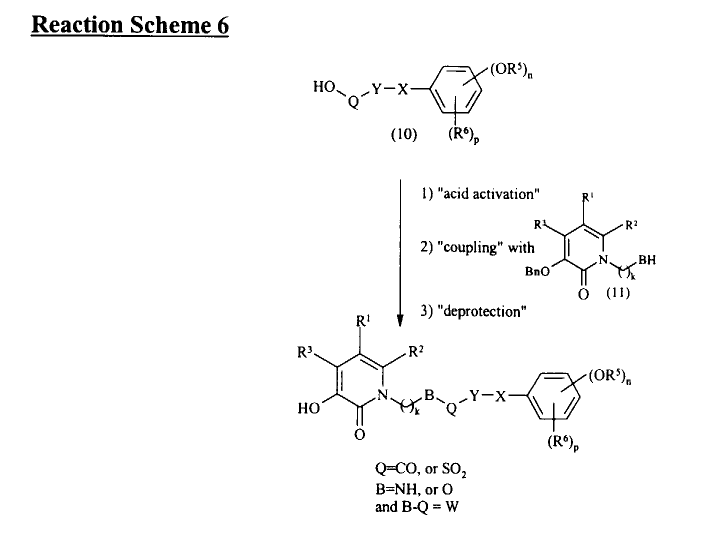

The primary amine or alcohol of the protected hydroxy pyridinone (11) can be prepared using standard synthetic chemistry.

The acid derivatives (carboxylic or sulfonic) of the respective antioxidants, (10) can be prepared using standard synthetic chemistry.

A specific method for the synthesis of compounds where q = 1, A is a 3-hydroxy-4-(1*H*)-pyridinone moiety and Z is (CH₂)ₖWY where W is a NH unit comprises reductive amination of the aldehyde of the respective antioxidant unit (12) with the primary amine of the protected hydroxy pyridinone (13) using standard conditions (for example NaCNBH₄ in MeOH), followed by removal of the benzyl protecting group, as illustrated in Reaction Scheme 7. (Alternatively, the reductive amination can be carried out in the reverse fashion using the aldehyde of the hydroxy pyridinone and the amine of the antioxidant unit.).

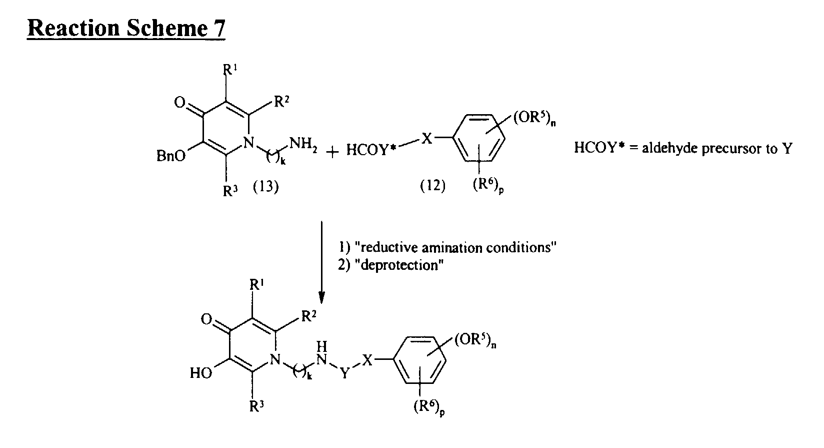

The primary amine of the protected hydroxy pyridinone (13) can be prepared using standard synthetic chemistry. (For example, for the synthesis of (13), where R¹ and R² are H, and R³ is methyl, k is 2 and 3, see Dobbin *et al.* J. Med. Chem., 1993, 36, 2448-58).

The aldehydes of the respective antioxidants (12) can be prepared using standard synthetic chemistry.

A second specific method for the synthesis of compounds where q = 1, A is a 3-hydroxy-4-(1*H*)-pyridinone moiety and Z is (CH₂)ₖWY where W is a NH unit comprises condensation of the bis-amine of the respective antioxidant unit (14) with a 3-benzyloxy-4-pyrone (2), followed by removal of the benzyl protecting group, as illustrated in Reaction Scheme 8.

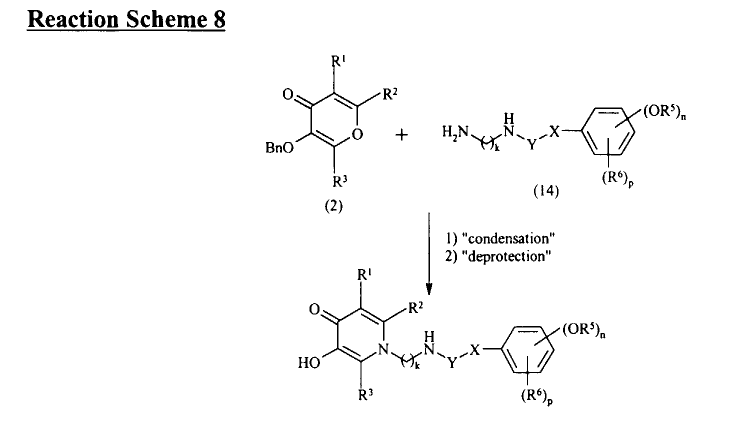

Bis-amine (14) can be prepared via standard methods. For example via reductive amination of the aldehyde derivative of the respective antioxidant unit (12) with di-aminoalkane (15) (using for example NaCNBH₄ in MeOH), followed by deprotection (using for example trifluoroacetic acid). Alternatively (14) can be formed via amidation of the acid derivative of the respective antioxidant unit (16) with di-aminoalkane (17), followed by deprotection, and finally reduction (using for example LiAlH₄). See Reaction Scheme 9.

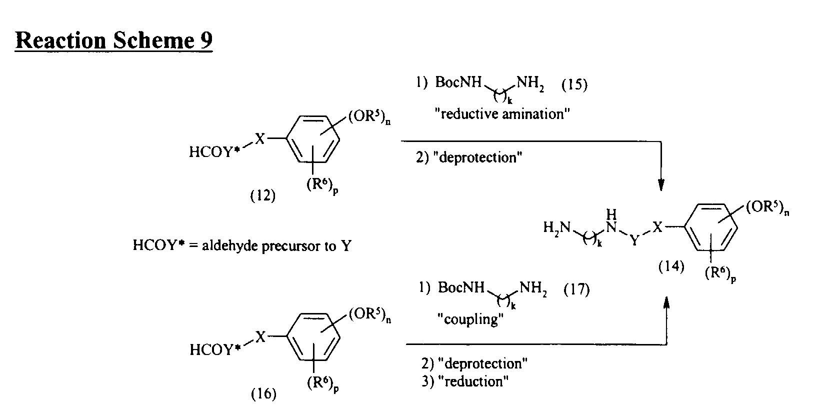

A specific method for the synthesis of compounds where q = 1, A is a 3-hydroxy-4-(1*H*)-pyridinone moiety and Z is (CH₂)ₖWY where W is a O atom comprises alkylation of the alcohol of the respective antioxidant unit (18) with an alkylating agent containing a nitrogen functionality (or a functionality capable of transformation into a amine group), for example compound (19), conversion of the product into a amine (using for example LiAlH₄), condensation of the amine with a 3-benzyloxy-4-pyrone (2), followed by removal of the benzyl protecting group. See Reaction Scheme 10.

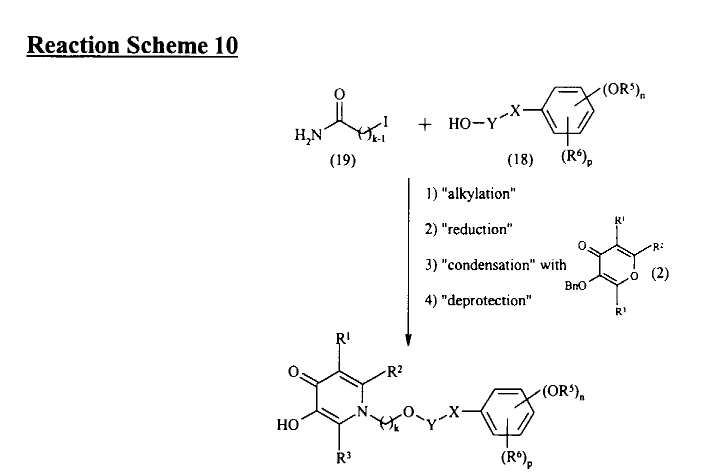

Compounds of the present invention which contain more than one hydroxy-pyridinone Fe-chelating unit (where q = 2 or 3) may be prepared via a general method, using standard synthetic chemistry. For example, compounds where q = 2 can be prepared via condensation of the bis-primary amine of the respective antioxidant unit (20) with two equivalents of a 3-benzyloxy-4-pyrone (2) (in the presence of base, for example NaOH in a MeOH:water solution), followed by removal of the benzyl protecting group (with for example H₂ and Pd/C), as illustrated in Reaction Scheme 11. Likewise, compounds where q = 3 can be prepared via condensation of a tris-primary amine of the respective antioxidant unit with three equivalents of a 3-benzyloxy-4-pyrone (2). (For the reaction of 3-benzyloxy-2-methyl-4-pyrone with 1,6-diaminohexane see Orvig *et al.,* Can. J. Chem., 1988, 66, 123-131).

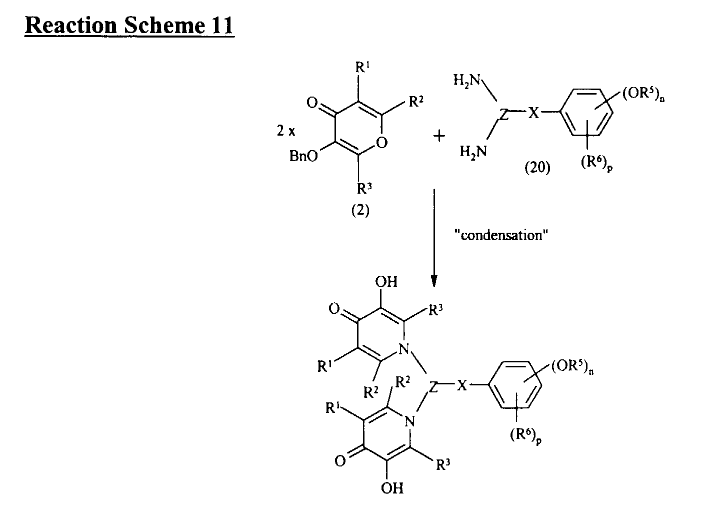

The bis-primary amine (20), (and tris-primary amines) may be prepared using standard synthetic chemistry.

A specific method of synthesis of compounds of the present invention which contain more than one hydroxy-pyridinone Fe-chelating unit, (where q = 2 or 3) and Z is (CH₂)ₖWY where W is a NHCO, OCO, NHSO₂ or OSO₂ unit, exists. For example, synthesis of compounds where q = 2 comprises coupling two equivalents of the primary amine or alcohol derivative of the protected hydroxy pyridinone (9) with the di-carboxylic or di-sulfonic acid derivative of the respective antioxidant unit (21) (using standard coupling techniques, for example via acid activation as a succinimdyl ester, or with coupling reagents such as EDCI), followed by removal of the benzyl protecting group. See Reaction Scheme 12. Likewise, compounds where q = 3 can be prepared via coupling a tris-carboxylic acid of the respective antioxidant unit with three equivalents of the primary amine or alcohol derivative of the protected hydroxy pyridinone (9).

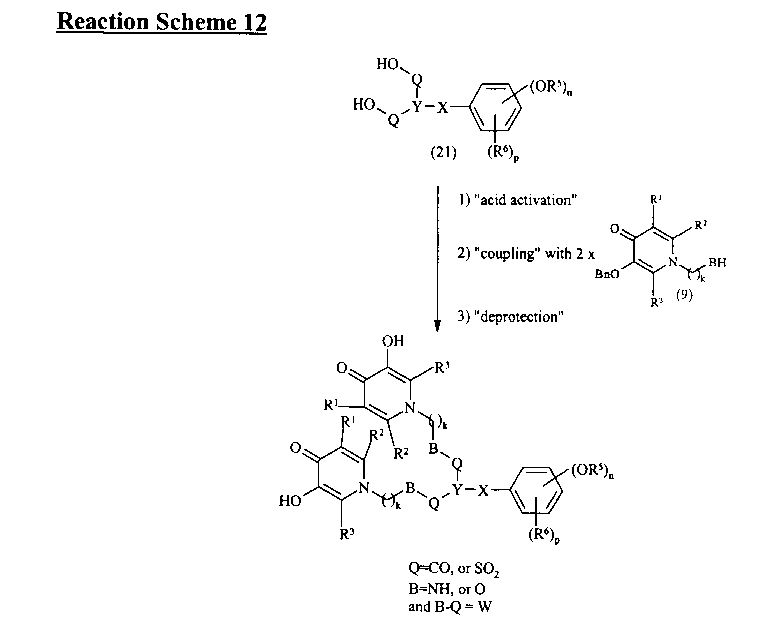

The di-carboxylic acid derivative of the antioxidant unit (21) can be prepared from bromide (22) via reaction with dimethylmalonate (in the presence of a suitable base, for example NaH) to produce the dimethylester, followed by saponification (using for example NaOH), as illustrated in Reaction Scheme 13.

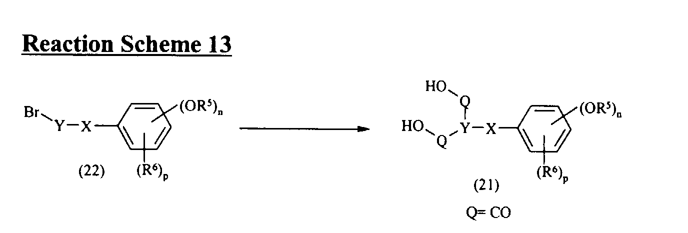

Phenolic -OH groups of the antioxidant unit starting materials/precursors, described above, can be optionally protected during synthetic manipulation (for example, as a benzyl ether). Deprotection to reveal the phenolic -OH groups of the antioxidant unit can be carried out simultaneously with removal of the protecting group of the hydroxy pyridinone unit, in the last step of the sequence.

The compounds of the present invention may contain one or more asymmetric carbon atoms, so that the compounds exist in different stereoisomeric forms. The compounds can be, for example, racemates or optically active forms. The optically active forms can be obtained by resolution of the racemates or by asymmetric syntheses.

The compounds of the present invention may also be in a prodrug form wherein some or all of the free -OH groups of the preferred compounds are derivatised (for example, via an ester, amide or phosphate bond) with a suitable group (the group may contain, for example, an alkyl, aryl, phosphate, sugar, amine, glycol, sulfonate or acid function) which is suitably labile so as it will be removed / cleaved (eg. by hydrolysis) to reveal the preferred compound sometime after administration or when exposed to the desired biological environment. Prodrug forms of the compounds of the present invention can be used to increase compound solubility and stability for formulation, increase efficacy and decrease metabolism.

Preferably the prodrug forms of the compounds of the present invention are ester linked alkyl or aryl amines. For example, esters derived from glycine, N-methyl glycine, N,N-dimethyl glycine, β-alanine, γ-aminobutyric acid, valine, or aminobenzoic acid.

Preferably the ester linked amine prodrug is derived from N,N-dimethyl glycine.

The prodrugs of the compounds of the present invention may be prepared using conventional synthetic methods. For example, a general method for synthesis of the preferred prodrugs of the compounds of the present invention comprises coupling a N-protected amino acid derivative with the hydroxy group of the antioxidant unit (using for example potassium carbonate in acetone), followed by deprotection. This is illustrated in Scheme 14 using a N-hydroxysuccinimide ester or an acid chloride as an example of the N-protected amino acid derivative.

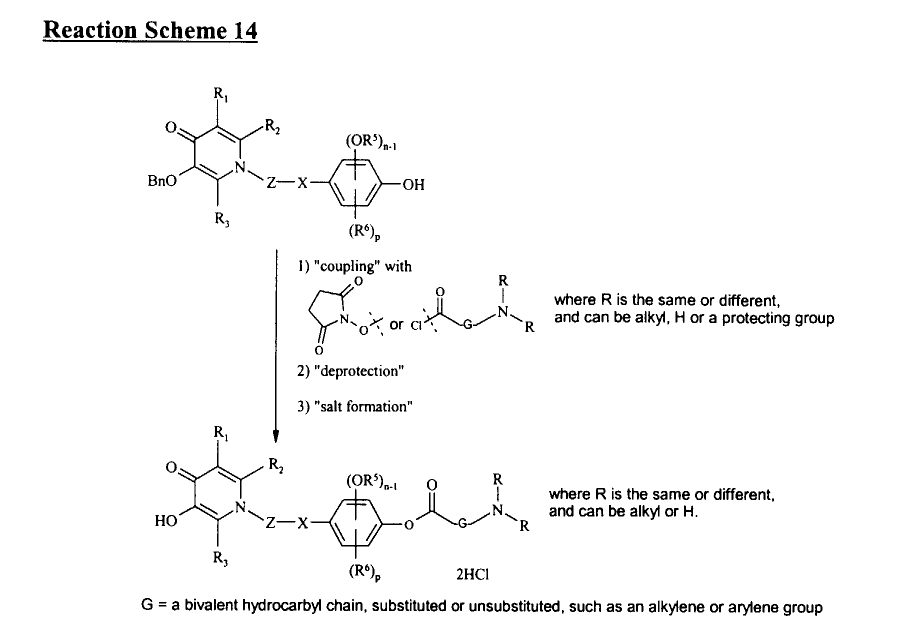

Alternatively, the N-protected amino acid derivative can be coupled to the hydroxy group of the pyridone unit, followed by deprotection, as illustrated in Scheme 15.

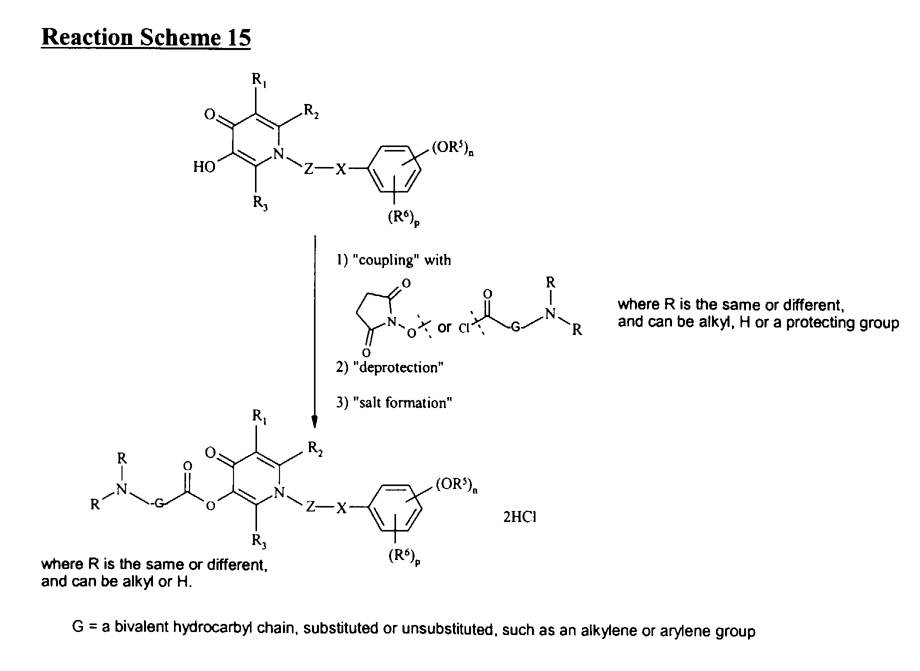

Schemes 14 and 15 illustrate the preparation of prodrugs of the compounds of the present invention where A = AI and q = 1. However, the synthetic routes shown in Schemes 14 and 15 are generally applicable to the preparation of prodrugs of any of the compounds of the present invention, i.e. where A = AI or All and q = 1, 2 or 3.

To further increase their efficacy, compounds of the present invention may also contain additional non-covalently linked components such as dextrans or cyclodextrins, which aid stability and dispersion, and decrease metabolism of the active ingredient.

According to a further aspect of the present invention there is provided the compounds of the present invention for use in therapy.

According to a further aspect of the present invention there is provided use of the compounds of the present invention in the manufacture of a medicament for the treatment of a condition associated with oxidative stress, particularly oxidative damage of the central nervous system.

The term "treatment" as used herein includes prophylaxis.

Diseases, disorders and medical treatments/procedures associated with oxidative stress that can be treated with the compounds of the present invention include: aging; acute intermittent porphyria; adriamycin-induced cardiomyopathy; AIDS dementia and HIV-1 induced neurotoxicity; Alzheimer's disease; atherosclerosis; cateract; cerebral ischaemia; cerebral palsy; cerebral tumour; chemotherapy-induced organ damage; cisplatin-induced nephrotoxicity; coronary artery bypass surgery; diabetic neuropathy; Down's syndrome; drowning; epilepsy and post-traumatic epilepsy; Friedrich's ataxia; frontotemporal dementia; glaucoma; glomerulopathy; haemochromatosis; haemodialysis; haemolysis; haemolytic uraemic syndrome (Weil's disease); haemorrhagic stroke; heart attack and reperfusion injury; Huntington's disease; Lewy body disease; intermittent claudication; ischaemic stroke; inflammatory bowel disease; macular degeneration; malaria; methanol-induced toxicity; meningitis (aseptic and tuberculous); motor neuron disease; multiple sclerosis; multiple system atrophy; myocardial ischaemia; neoplasia; Parkinson's disease; peri-natal asphyxia; Pick's disease; progressive supra-nuclear palsy; radiotherapy-induced organ damage; restenosis after angioplasty; retinopathy; senile dementia; schizophrenia; sepsis; septic shock; spongiform encephalopathies; subharrachnoid haemorrage/cerebral vasospasm; subdural haematoma; surgical trauma, including neurosurgery; thalassemia; transient ischaemic attack (TIA); traumatic brain injury (TBI); traumatic spinal injury; transplantation; vascular dementia; viral meningitis; and viral encephalitis.

Additionally, compounds of the present invention may also be used to potentiate the effects of other treatments, for example to potentiate the neuroprotective effects of brain derived nerve growth factor.

The invention is particularly directed to conditions which induce oxidative damage of the central nervous system, including acute and chronic neurological disorders such as traumatic brain injury, spinal cord injury, cerebral ischaemia, stroke (ischaemic and haemorragic), subharrachnoid haemorrage/cerebral vasospasm, cerebral tumour, Alzheimer's disease, Huntington's disease, Parkinson's disease, Friedrich's ataxia, motor neuron disease and multiple sclerosis.

The invention further provides a method of treating a condition associated with oxidative stress, particularly oxidative damage of the central nervous system, comprising administering to a patient in need of such treatment an effective dose of a compound of the present invention.

The invention further provides a pharmaceutical composition comprising a compound of the present invention in combination with a pharmaceutically acceptable carrier or excipient and a method of making such a composition comprising combining a compound of the present invention with a pharmaceutically acceptable carrier or excipient.

Compounds of the present invention may be administered in a form suitable for oral use, for example a tablet, capsule, granule, powder, aqueous or oily solution, suspension or emulsion; for topical use including transmucosal and transdermal use, for example a cream, ointment, gel, aqueous or oil solution or suspension, salve, patch or plaster; for nasal use, for a example a snuff, nasal spray or nasal drops; for vaginal or rectal use, for example a suppository; for administration by inhalation, for example a finely divided powder or a liquid aerosol; for sub-lingual or buccal use, for example a tablet or capsule; or for parenteral use (including intravenous, subcutaneous, intramuscular, intravascular, intrathecal or infusion), for example a sterile aqueous or oil solution or suspension. In general the above compositions may be prepared in a conventional manner using conventional excipients, using standard techniques well known to those skilled in the art of pharmacy. Preferably, the compound is administered orally for chronic disorders such as Alzheimer's and Parkinson's disease, and intravenously for acute disorders such as stroke and TBI.

For oral administration, the compounds of the invention will generally be provided in the form of tablets or capsules or as an aqueous solution or suspension.

Tablets for oral use may include the active ingredient mixed with pharmaceutically acceptable excipients such as inert diluents, disintegrating agents, binding agents, lubricating agents, sweetening agents, flavouring agents, colouring agents and preservatives. Suitable inert diluents include sodium and calcium carbonate, sodium and calcium phosphate, cyclodextrin, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

Capsules for oral use include hard gelatin capsules in which the active ingredient is mixed with a solid diluent, and soft gelatin capsules wherein the active ingredient is mixed with water or an oil such as peanut oil, liquid paraffin or olive oil.

For intramuscular, intraperitoneal, subcutaneous and intravenous use, the compounds of the invention will generally be provided in sterile aqueous solutions or suspensions, buffered to an appropriate pH and isotonicity. Suitable aqueous vehicles include Ringer's solution and isotonic sodium chloride. Aqueous suspensions according to the invention may include suspending agents such as cellulose derivatives, lipids, sodium alginate, polyvinylpyrrolidone, cyclodextrins, gum tragacanth, polyethylene glycol, propylene glycol, N,N-dimethylacetamide, cremophors, polysorbates, liposomes and wetting agents such as lecithin. Suitable preservatives for aqueous suspensions include ethyl and n-propyl *p*-hydroxybenzoate.

It will be appreciated that the dosage levels used may vary over quite a wide range depending upon the compound used, the severity of the symptoms exhibited by the patient and the patient's body weight.

The invention will now be described in detail with reference to the following examples. It will be appreciated that the invention is described by way of example only and modification of detail may be made without departing from the scope of the invention.

### EXPERIMENTAL

### Synthetic Examples

### Example 1

**3-(1,4-Dihydro-3-hydroxy-2-methyl-4-oxo-1-pyridinyl)-N-(2-(5-methoxy-3-indolyl)ethyl)propanamide mesylate**

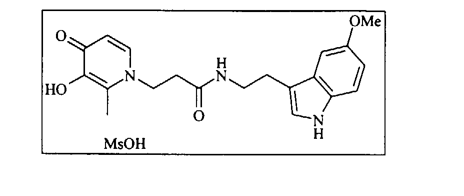

*3-(3-Benzyloxy-1,4-dihydro-2-methyl-4-oxo-1-pyridinyl)-N-(2-(5-methoxy-3-indolyl)ethyl)propanamide*

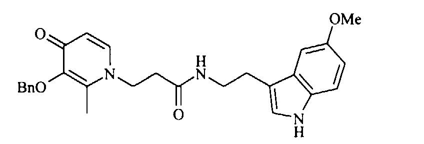

Succinimidyl 3-(3-benzyloxy-1,4-dihydro-2-methyl-4-oxo-1-pyridinyl)propanoate [Dobbin *et al.* J. Med. Chem., (1993), 36, 2448-2458] (6.1 g, 15.9 mmol), 5-methoxytryptamine (3.0 g, 15.9 mmol) and CH₂Cl₂ (120 mL) were stirred at room temperature for 20 h, extracted with 2.0-M NaOH, dried (Na₂SO₄), concentrated *in vacuo* and purified by chromatography [SiO₂; CH₂Cl₂-MeOH (90:10)] to give the *product* (6.7 g, 90 %) as a white solid: mp 67-72 °C; IR νₘₐₓ (Nujol)/cm⁻¹ 3261, 2929, 1652, 1557, 1252, 1218, 736 and 702; NMR δ_{H} (400 MHz, CDCl₃) 2.16 (3H, s), 2.53 (2H, t, *J* 6.2 Hz), 2.86 (2H, t, *J* 6.6 Hz), 3.53 (2H, q, *J* 6.5 Hz), 3.8 (3H, s), 4.09 (2H, t, *J*6.4 Hz), 5.08 (2H, s), 6.38 (1H, d, *J*7.3 H), 6.78 (1H, dd, *J* 8.8, 2.2 Hz), 6.81 (1H, s), 6.94 (1H, d, *J* 2.2 Hz), 7.1 (1H, br s), 7.21 (1H, d, *J* 8.8 Hz), 7.28-7.35 (6H, m) and 8.58 (1H, s); Anal. Calcd. for C₃₀H₃₉NO₃ · H₂O: C, 67.91; H, 6.54; N, 8.80. Found: C, 68.08; H, 6.26; N, 8.74.

*3-(1,4-Dihydro-3-hydroxy-2-methyl-4-oxo-1-pyridinyl)-N-(2-(5-methoxy-3-indolyl)ethyl)propanamide*

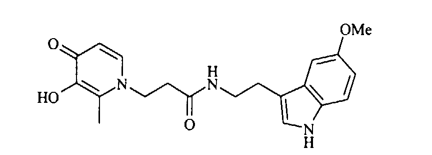

3-(3-Benzyloxy-1,4-dihydro-2-methyl-4-oxo-1-pyridinyl)-N-(2-(5-methoxy-3-indolyl)ethyl)propanamide (6.34 g, 13.8 mmol) and 10 % Pd/C (2.5 g) in EtOH (220 mL) was stirred under a H₂ atmosphere for 20 h. The mixture was filtered through Celite^{®}, concentrated *in vacuo,* and purified by chromatography [Sephadex^{®} LH-20; CH₂Cl₂-MeOH (90:10)] to give the *product* (4.08 g, 84 % yield) as a white solid: mp 104-109 °C; IR νₘₐₓ (Nujol)/cm⁻¹ 3223, 1629, 1564, 1508, 1219, 1068, 1037 and 817; NMR δ_{H} (400 MHz, DMSO-*d*₆) 2.30 (3H, s), 2.51 (2H, t, *J* 7.3Hz), 2.72 (2H, t, *J* 7.3 Hz), 3.30 (2H, q, *J* 6.5 Hz), 3.76 (3H, s), 4.15 (2H, t, *J*6.8 Hz), 6.08 (1H, d, *J* 7.3 Hz), 6.7 (1H, dd, *J* 8.8, 1.8 Hz), 7.00 (1H, s), 7.07 (1H, s), 7.22 (1H, d, *J* 8.8 Hz), 7.46 (1H, d, *J* 7.3 Hz), 8.09 (1H, t, *J* 5.2 Hz), and 10.64 (1H, s); Anal. Calcd. for C₂₀H₂₃N₃O₄ · 0.5H₂O: C, 63.48; H, 6.39; N, 11.10. Found: C, 63.36; H, 6.54; N, 10.81.

*3-(1,4-Dihydro-3-hydroxy-2-methyl-4-oxo-1-pyridinyl)-N-(2-(5-methoxy-3-indolyl)ethyl)propanamide mesylate*

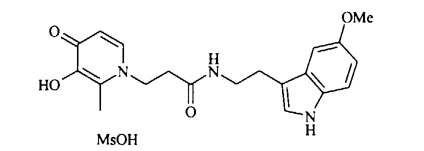

Methanesulphonic acid (176 µL, 2.7 mmol) was added dropwise to 3-(1,4-dihydro-3-hydroxy-2-methyl-4-oxo-1-pyridinyl)-N-(2-(5-methoxy-3-indolyl)ethyl)propanamide (1.0 g, 2.7 mmol) in Et₂O (50 mL) and CH₂Cl₂ (60 mL). The mixture was stirred for 1.5 h, concentrated *in vacuo,* suspended in CHCl₃ and the solid collected by filtration to give the *title compound (1.2* g, 94 % yield) as a mauve solid: mp 70 °C (dec); IR νₘₐₓ (Nujol)/cm⁻¹ 3593, 3248, 2530, 1633, 1219, 1149, 775, 553, and 526; NMR δ_{H} (400 MHz, DMSO-*d*₆) 2.35(3H, s), 2.55(3H, s), 2.69 (2H, pent, *J* 7.5 Hz), 3.29 (2H, q, *J* 7.5 Hz), 3.76 (3H, s), 4.55 (2H, t, *J* 6.8 Hz), 6.72 (1H, dd, *J*8A, 2.2 Hz),6.99 (1H, d, *J* 1.8 Hz), 7.08 (1H, s), 7.11 (1H, d, *J* 6.8 Hz), 7.22 (1H, d, *J* 8.4 Hz), 8.14 (1H, d, *J* 6.8 Hz), 8.15 (1H, br s), and 10.65 (1H, s); Anal. Calcd. for C₂₁H₂₇N₃O₇S: S, 6.89. Found: S, 7.05.

### Example 2

**3-Hydroxy-1-(2-(5-methoxy-3-indolyl)ethyl)-2-methyl-4(1*H*)-pyridinone**

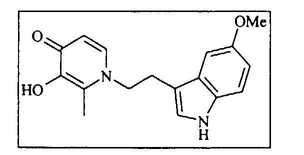

*3-Benzyloxy-1-(2-(5-methoxy-3-indolyl)ethyl)-2-methyl-4(1H)-pyridinone*

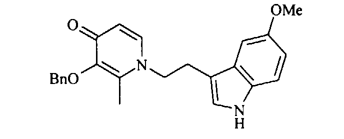

A solution of 5-methoxytryptamine (1.03 g, 5.41 mmol), 3-benzyloxy-2-methyl-4-pyrone [Dobbin *et al.* J. Med. Chem. (1993), 36(17), 2448-58] (1.17 g, 5.41 mmol) and 5.0-M NaOH (5 mL) in EtOH (50 mL) and H₂O (50 mL) were refluxed for 2 h. The mixture was cooled, concentrated *in vacuo* and the residue partitioned between CH₂Cl₂ and H₂O. The organic layer was separated, dried MgSO₄), concentrated *in vacuo* and the crude product purified by chromatography [SiO₂; CH₂Cl₂-MeOH-NH₄OH (95:5:1)] to give *the product* (0.89 g, 42 %) as a pale-yellow foam: mp softens over 60-65 °C; IR νₘₐₓ (Nujol)/cm⁻¹ 2922, 1624, 1554, 1463, 1377, 1247, 1216, 828 and 702; NMR δ_{H} (400 MHz, CDCl₃) 1.99 (3H, s), 3.01 (2H, t, *J* 6.6 Hz), 3.83 (3H, s), 4.00 (2H, t, *J* 6.6 Hz), 5.18 (2H, s), 6.21 (1H, d, *J* 7.6 Hz), 6.62 (1H, d, *J* 2.5 Hz), 6.80-6.86 (3H, m), 7.24-7.39 (6H, m) and 8.59 (1H, br s); m/z (ES+) 389 (100 %), 390 (34).

*3-Hydroxy-1-(2-(5-methoxy-3-indolyl)ethyl)-2-methyl-4(1H)-pyridinone*

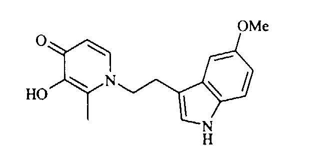

A solution of 3-benzyloxy-1-(2-(5-methoxy-3-indolyl)ethyl)-2-methyl-4(1*H*)-pyridinone (0.80 g, 2.06 mmol) in MeOH (50 mL), containing 10 % Pd-C (0.10 g) was stirred vigorously under a H₂ atmosphere for 17 h. The mixture was then filtered through Celite^{®}, washed through with warm MeOH, and the filtrate concentrated *in vacuo* to give the *title compound* (0.62g, 100 %) as a pink solid: mp darkens 175 °C, melts 184-185 °C; IR νₘₐₓ (Nujol)/cm⁻¹ 3188, 2925, 1628, 1569, 1511, 1464, 1259, 1078 and 801; NMR δ_{H} (400 MHz, DMSO-*d*₆) 2.24 (3H, s), 3.03 (2H, t, *J* 7.0 Hz), 3.72 (3H, s), 4.13 (2H, t, *J* 7.1 Hz), 6.02 (1H, d, *J* 7.4 Hz), 6.69 (1H, dd, *J* 8.4, 2.0 Hz), 6.94 (1H, d, *J* 2.3 Hz), 7.05 (1H, s), 7.21 (1H, d, *J* 8.3 Hz), 7.43 (1H, d, *J* 7.2 Hz) and 10.73 (1H, br s); Anal.Calcd. for C₁₇H₁₈N₂O₃ · 0.5 H₂O: C, 66.43; H, 6.23; N, 9.11. Found: C, 66.82; H, 6.08; N, 9.20.

### Example 3

**2-(1,2-Dihydro-3-hydroxy-2-oxo-1-pyridinyl)-N-(2-(5-methoxy-3-indolyl)ethyl)acetamide**

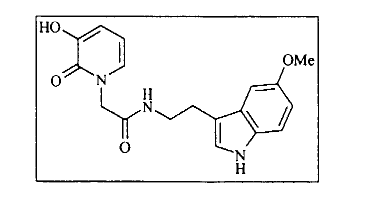

*2-(3-Benzyloxy-1,2-dihydro-2-oxo-1-pyridinyl)-N-(2-(5-methoxy-3-indolyl)ethyl)acetamide*

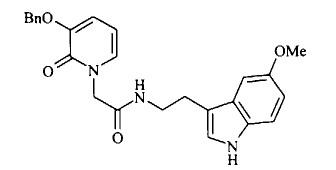

This is prepared from succinimidyl 3-(3-benzyloxy-1,2-dihydro-2-oxo-1-pyridinyl)acetate [Streater *et al.* J. Med. Chem. (1990), 33(6), 1749-1755] and 5-methoxytryptamine according to the method described in Example 1 to give the *product.*

*2-(1,2-Dihydro-3-hydroxy-2-oxo-1-pyridinyl)-N-(2-(5-methoxy-3-indolyl)ethyl)acetamide*

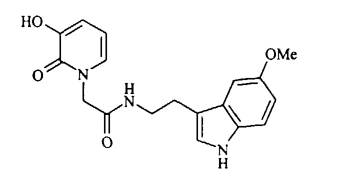

This is prepared from 2-(3-benzyloxy-1,2-dihydro-2-oxo-1-pyridinyl)-N-(2-(5-methoxy-3-indolyl)ethyl)acetamide according to the method described in Example 1 to give the *title compound.*

### Example 4

**N-(2-(1,4-dihydro-3-hydroxy-2-methyl-4-oxo-1-pyridinyl)ethyl)-2-(2-(1,4-dihydro-3-hydroxy-2-methyl-4-oxo-1-pyridinyl)ethylaminocarbonyl)-4-(5-methoxy-3-indolyl)butanamide**

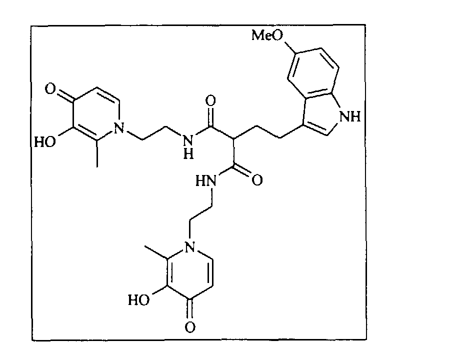

*Methyl 2-methoxycarbonyl-4-(5-methoxy-3-indolyl)butanoate*

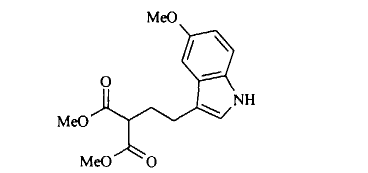

A suspension of hexane-washed NaH (60 % dispersion in mineral oil, 73 mmol) in a mixture of THF (80 mL) and DMF (30 mL) is cooled to 0 °C under Ar and treated dropwise with dimethyl malonate (69 mmol). After a further 10 min at 20 °C, a solution of 3-(2-bromoethyl)-5-methoxy indole [Miettinen *et al.* Planta Med. (1996), 62, 42-5] (34 mmol) in a mixture of THF (20 mL) and DMF (20 mL) is added, and the mixture is heated under gentle reflux for 1 h. The reaction is then poured into brine, extracted with EtOAc and concentrated *in vacuo.* Kugelrohr distillation at 130 °C (20mm Hg) is used to remove unreacted dimethyl malonate. The residue is purified by chromatography [SiO₂; EtOAc-Hexane] to give the *product.*

*2-Carboxy-4-(5-methoxy-3-indolyl)butanoic acid*

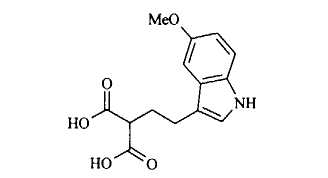

A solution of methyl 2-methoxycarbonyl-4-(5-methoxy-3-indolyl)butanoate (60.0 mmol) in a mixture of THF (80 mL) and water (30 mL) is treated with NaOH (130 mmol). After stirring at room temperature for 24 h the reaction is then concentrated *in vacuo.* The residue is acidified to pH 4-5, and the aqueous phase is extracted with EtOAc. The organic layer is washed with brine, dried (MgSO₄), and purified by chromatography [SiO₂; EtOAc-Hexane] to give the *product.*

*N-(2-(3-Benzyloxy-1,4-dihydro-2-methyl-4-oxo-1-pyridinyl)ethyl)-2-(2-(3-benzyloxy-1,4-dihydro-2-methyl-4-oxo-1-pyridinyl)ethylaminocarbonyl)-4-(5-methoxy-3-indolyl)butanamide*

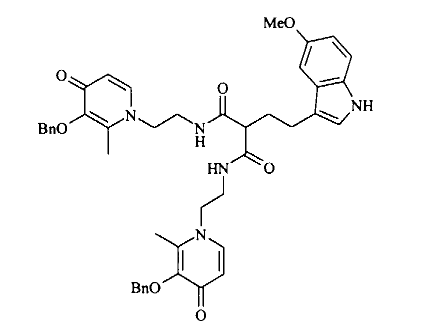

A solution of 2-carboxy-4-(5-methoxy-3-indolyl)butanoic acid (4.0 mmol), 1-(2-aminoethyl)-3-benzyloxy-2-methyl-4(1*H*)-pyridinone [Feng *et al.* J. Med. Chem. (1993), 36(19), 2822-7] (8.0 mmol), 1-ethyl-3-[3-(dimethylamino)propyl]carbodiimide hydrochloride (8.0 mmol) and DMAP (8.0 mmol) in THF (50 mL) is stirred at room temperature for 17 h. H₂O (20 mL) is added, the mixture is concentrated *in vacuo* and the residue is extracted with CH₂Cl₂ (2 x 20 mL). The organic phase is dried (MgSO₄), concentrated *in vacuo* and purified by chromatography [SiO₂; CH₂Cl₂-MeOH (95:5)] to give the *product.*

*N-(2-(1,4-dihydro-3-hydroxy-2-methyl-4-oxo-1-pyridinyl)ethyl)-2-(2-(1,4-dihydro-3-hydroxy-2-methyl-4-oxo-1-pyridinyl)ethylaminocarbonyl)-4-(5-methoxy-3-indolyl)butanamide*

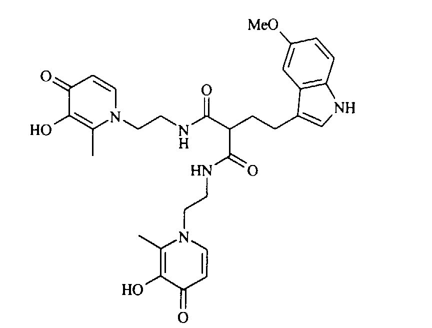

This is prepared from N-(2-(3-benzyloxy-1,4-dihydro-2-methyl-4-oxo-1-pyridinyl)ethyl)-2-(2-(3-benzyloxy-1,4-dihydro-2-methyl-4-oxo-1-pyridinyl)ethylaminocarbonyl)-4-(5-methoxy-3-indolyl)butanamide according to the method described in Example 1 to produce the *title compound.*

### Example 5

**1-(2-(5-methoxy-3-indolyl)ethyl)-2-methyl-4(1*H*)-oxopyridin-3-yl-N,N-dimethylaminoacetate dihydrochloride**

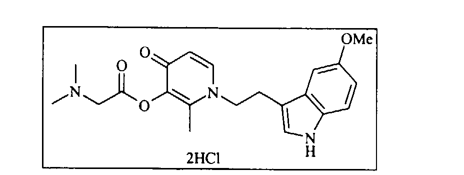

To a stirred solution of 3-hydroxy-1-(2-(5-methoxy-3-indolyl)ethyl)-2-methyl-4(1*H*)-pyridinone (1.84 mmol) in dry pyridine (10 mL) under argon at 0°C is added *N,N*-dimethylglycyl chloride hydrochloride (0.58 g, 3.68 mmol). The suspension is stirred under argon at room temperature for 24 h and then heated at 70°C for 48 h. After cooling, the reaction mixture is evaporated *in vacuo.* The residue is partitioned between CH₂Cl₂ (40 mL) and water (40 mL). The organic phase is separated, washed with water (30 mL) and brine (30 mL), dried (MgSO₄) and evaporated *in vacuo.* The crude material is purified by chromatography [SiO₂; CHCl₃:MeOH:AcOH (90:8:2)] to give the *product* as the free base. This is then treated according to the method described in Example 1 using 1.0M-HCl in Et₂O to produce the *title compound.*

### Biological Testing Procedures and Data

The ability of the compounds of the present invention to protect against oxidative damage has been shown in both *in vitro* and *in vivo* models. These models are briefly explained below.

### Lipid Peroxidation assay

Lipid peroxidation in rat brain homogenates is a general procedure used to measure the antioxidant capacity of molecules in a biological environment (Das N.P. and Ratty A.K., Biochem. Med. Metab. Biol, 1987, 37, 256-264). Compounds of the present invention have been shown to be potent inhibitors of lipid peroxidation.

### Iodoacetate induced cell toxicity in culture

Iodoacetate, via its alkylation of glyceraldehyde-3-phosphate dehydrogenase (GAPDH), is a potent inhibitor of glycolysis and hence energy production in cells. This form of chemical hypoxia has been demonstrated to result in a state of oxidative stress, from which the cells can be rescued by anti-oxidant molecules (Uto A. *et al.,* J. Neurochem. 1995, 64, 2185-2192; Malcolm C.S. *et al.,* Soc. Neurosci. Abstr. 1998). The compounds of the present invention have been shown to protect cerebellar granule cells from this chemical induced oxidative stress.

### Malonic Acid lesion model

Malonic acid is a competitive inhibitor of succinate dehydrogenase. It depletes extracellular ATP production, and intrastriatal injection has previously been demonstrated to cause a NMDA receptor mediated lesion (Greene *et al.,* J. Neurochem., 1993, 61, 1151-1154). The neuroprotective ability of compounds of the present invention are assessed in this animal model of oxidative stress.

### 1. In Vitro Assays

### 1a. Lipid Peroxidation

### Procedure

Rat cortex was homogenised in 20 vols. of ice cold 0.32-M sucrose and centrifuged at 1,000 g for 10 min. The pellet was discarded whilst the resulting supernatant was centrifuged at 15,000 g for 20 min at 4 °C to yield p₂ pellets. The pellet was re-suspended in ice-cold 50.0-mM phosphate buffer and centrifuged at 30,000 g for 20 min at 4 °C. The pellet was re-suspended in 30 vols. of 50.0-mM phosphate buffer and used immediately in the assay.

Assays contained 500-µM L-ascorbic acid to induce lipid peroxidation, plus various concentrations of test compound, with the tissue preparation in a total volume of 500 µL. This was incubated at 37 °C for 30 min. Lipid peroxidation was measured by adding to the reaction mixture, 300 µL 40 % (w/v) trichloroacetic acid, 150 µL 5.0-M HCl and 300 µL 2 % (w/v) 2-thiobarbituric acid (TBA). Samples were then heated at 90 °C in a water bath for 15 min, and centrifuged at 15,000 g for 10 min. The pink colour of the supernatant was assessed spectrophotometrically at a wavelength of 532 nm. The amount of malondialdehyde (MDA) in the samples was calculated using a standard curve prepared with malondialdehyde tetrabutylammonium salt (Das, N.P. and Ratty, A.K. Biochem. Med. Metab. Biol. (1987), 37, 256-264).

### Data

Results are expressed as IC₅₀ values (µM) and presented in Table 1. The IC₅₀ values show the concentration of test compound required to inhibit the lipid peroxidation by 50%.

**Table 1**

| **Lipid Peroxidation** | |
|---|---|
| **Compound Example No**. | **IC**_{**50**} **(µM)** |
| Example 1 | 1.5 |
| Example 2 | 2.0 |

The results demonstrate that the compounds of the present invention inhibit the peroxidation of lipid membranes induced by ascorbic acid.

### 1b. Cell Death

### Procedure

After 6-8 days in culture, cerebellar granule cell (CGC) cultures in 96-well plates (250,000 cells per well) were prepared for hypoglycaemia. lodoacetate (IAA) was made up in a balanced salt solution (BSS) (NaCl, 154.0-mM; KCl, 5.6-mM; MgCl₂, 1.0-mM; CaCl₂, 2.5-mM; N-[2-hydroxyethyl]piperazine-N'-[2-ethanesulfonic acid] (HEPES), 10.0-mM; D-glucose, 5.6-mM; pH 7.4) and any neuroprotective agents were made up in pre-warmed tissue culture media and allowed to equilibrate in a controlled environment (5 % CO₂, 95 % air). The assay was initiated by aspiration of the maintenance media, which was replaced by either BSS (control) or 30-µM IAA in BSS, both solutions containing 10-µM of the NMDA receptor antagonist MK-801. The exposure to IAA was for 30 min only at 37 °C, after which time the BSS was aspirated and replaced with fresh, pre-equilibriated maintenance media containing various concentrations of test compound. All conditions were performed at least in duplicate in each 96 well plate. The final volume for each well was always 200 µL. Following a 24 hour incubation, visual inspection of the cells was followed by quantification of neuronal cell death by measuring 3-[4,5-dimethylthiazol-2-yl]-2,5-diphenyltetrazolium bromide (MTT)-reductase activity as described previously (Malcolm *et al.,* J. Neurochem. 1996, 66, 2350-2360).

### Data

Results are expressed as EC₅₀ values (µM), and presented in Table 2.

**Table 2**

| **Cell Toxicity** | |
|---|---|
| **Compound** | **EC**_{**50**} **(µM)** |
| Example 1 | 4.9 |
| Example 2 | 11.9 |

The results demonstrate the protective effect of the compounds of the invention on neuronal cells exposed to oxidative damage induced by IAA.

### 2. In Vivo Assay

### Malonic Acid Lesion Model

### Procedure

Malonic acid is a competitive inhibitor of succinate dehydrogenase, a key enzyme in both the tricarboxylic acid cycle and oxidative phosphorylation. Injection of malonic acid into the striatum causes ATP depletion, resulting in an excitotoxic lesion (Greene *et al.,* J. Neurochem., 1993, 61, 1151-1154). 2 µL of a 0.5-M malonic acid solution is injected into the right striatum of rats, with or without test compounds. 24 hours after surgery the animals are sacrificed and the size of the lesion measured using TTC histochemistry.

## Claims

1. A compound of the formula (1): 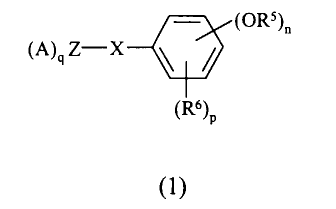 wherein A is 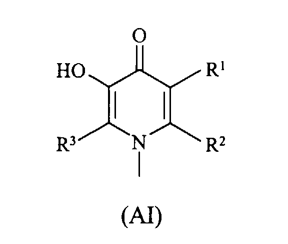 or 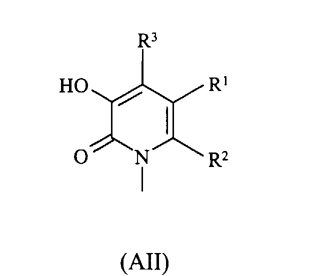
wherein R¹, R² and R³ are independently selected from H and alkyl;
wherein X is O, S, NR⁴ or a direct bond, wherein R⁴ is H or alkyl;
wherein Z is a hydrocarbyl chain comprising from 2 to 10 carbon atoms and one or more carbon-carbon double bonds, the hydrocarbyl chain also being optionally interrupted by one or more heteroatom(s) and optionally substituted by one or more oxo substituent(s);
wherein q is 1, 2 or 3;
wherein if q is 2 or 3, then each A can be the same or different;
wherein the or each R⁵ is independently selected from H and alkyl;
wherein the or each R⁶ is independently selected from alkyl;
wherein n is 1 to 5;
wherein p is 0 to 4; and
wherein the sum ofn and p is less than 6,
or a pharmaceutically acceptable salt thereof.

2. A compound according to claim 1 wherein R¹, R² and R³ are independently selected from H and unsubstituted alkyl.

3. A compound according to claim 1 wherein A is AI and R² and R³ are independently selected from H, unsubstituted alkyl, CH₂OR⁷, CH₂OCOR⁷, COOR⁷, CH₂NHR⁷, CH₂NHCOR⁷ and CONHR⁷ wherein R⁷ is selected from H and alkyl.

4. A compound according to claim 3 wherein R¹ is H or unsubstituted alkyl.

5. A compound according to claim 1 wherein A is AI and R¹ and R² are H and R³ is unsubstituted alkyl.

6. A compound according to claim 1, 2, 3, 4 or 5 wherein R³ is methyl.

7. A compound according to claim 1 wherein A is All and R¹, R² and R³ are independently selected from H, unsubstituted alkyl, CH₂OR⁷, CH₂OCOR⁷, COOR⁷, CH₂NHR⁷, CH₂NHCOR⁷ and CONHR⁷ wherein R⁷ is selected from H and alkyl.

8. A compound according to claim 1 wherein A is AII, and R¹, R² and R³ are H.

9. A compound according to any one of claims 1 to 8 wherein q = 1.

10. A compound according to any one of claims 1 to 9 wherein X is NR⁴ or a direct bond.

11. A compound according to any one of claims 1 to 9 where X is NR⁴ and the carbon-carbon double bond of the chain Z is immediately adjacent the NR⁴ group in the chain defined as Z-X.

12. A compound according to any one of claims 1 to 11 wherein X is NR⁴ and R⁴ is H.

13. A compound according to any one of the previous claims wherein R⁵ is H or C₁₋₁₀ alkyl.

14. A compound according to any one of the previous claims wherein R⁶ is methyl, isopropyl or t-butyl.

15. A compound according to any one of claims 1 to 12 wherein R⁵ is alkyl and is cyclised onto the chain Z.

16. A compound according to any one of claims 1 to 12 wherein R⁶ is alkyl and is cyclised onto the chain Z.

17. A compound according to any one of claims 1 to 12 wherein X is NR⁴, and Z is cyclized onto the phenyl ring shown in formula (1) to give a 5 or 6 membered heterocyclic ring containing a carbon-carbon double bond.

18. A compound according to any one of claims 1 to 17 wherein Z is a hydrocarbyl chain comprising from 2 to 10 carbon atoms and one or more carbon-carbon double bonds, the hydrocarbyl chain also being optionally interrupted by one or more group(s) selected from O, S, SO, SO₂, SO₂NH, NHSO₂, NH, NHCO, CONH, NHCONH, NHCOO, OCONH, OCO and COO.

19. A compound according to any one of claims 1 to 18 wherein Z is (CH₂)ₖWY wherein k is 0 to 10, wherein W is selected from O, S, SO, SO₂, SO₂NH, NHSO₂, NH, NHCO, CONH, NHCONH, NHCOO, OCONH, OCO, COO and a direct bond; wherein Y is a hydrocarbyl chain comprising from 2 to 10 carbon atoms and one or more carbon-carbon double bond(s); and wherein the chain (CH₂)ₖWY comprises from 2 to 10 carbon atoms.

20. A compound according to claim 19 wherein k is 1, 2, 3 or 4.

21. A compound according to claim 19 wherein when k is 0, W is selected from O, NHCO, NHCONH, NHCOO, OCONH and a direct bond; or when k is 1, W is selected from SO, SO₂, SO₂NH, CONH, COO and a direct bond.

22. A compound according to claim 19, 20 or 21 wherein Y is a hydrocarbyl chain comprising 3, 4 or 5 carbon atoms and one or more carbon-carbon double bond(s).

23. A compound according to any preceding claim wherein Z contains one carbon-carbon double bond.

24. A compound according to any one of claims 1 to 23 for use in therapy.

25. Use of a compound according to any one claims 1 to 23 in the manufacture of a medicament for the treatment of a condition associated with oxidative stress.

26. A method of treating a condition associated with oxidative stress comprising administering to a patient in need of such treatment an effective dose of a compound according to any one of claims 1 to 23.

27. A use or method according to claim 25 or 26 wherein said oxidative stress is oxidative damage of the central nervous system.

28. A use or method according to claim 25 or 26 wherein said condition is an acute or chronic neurological disorder.

29. A use or method according to claim 25 or 26 wherein said neurological disorder is traumatic brain injury, spinal cord injury, cerebral tumour, subharrachnoid haemorrage/cerebral vasospasm, cerebral ischaemia, stroke (ischaemia or haemorragic), Alzheimer's disease, Huntington's Disease, Parkinson's Disease, Friedrich's ataxia, motor neuron disease or multiple sclerosis.

30. A pharmaceutical composition comprising a compound according to any one of claims 1 to 23 in combination with a pharmaceutically acceptable carrier or excipient.
